# EUROPEAN PATENT APPLICATION

(11) **EP 1 121 912 A2**
(43) Date of publication of application: **08.08.2001**
(21) Application number: 01102006.2
(22) Date of filing: 30.01.2001
(51) Int. Cl.: A61F 7/08

(54) **Heating packet**

(30) Priority: 31.01.2000 JP 2000023070
(71) Applicant: JAPAN PIONICS CO., LTD., Minato-ku, Tokyo 105-0003 (JP)
(72) Inventor: Otsuka, Kenji, Japan Pionics Co., Ltd., Hiratsuka-shi, Kanagawa-ken (JP); Matsumoto, Yoshiki, Japan Pionics Co., Ltd., Hiratsuka-shi, Kanagawa-ken (JP); Yada, Koichi, Japan Pionics Co., Ltd., Hiratsuka-shi, Kanagawa-ken (JP); Ochi, Koshi, Japan Pionics Co., Ltd., Hiratsuka-shi, Kanagawa-ken (JP)
(74) Representative: Leifert, Elmar, Dr.

(57) **Abstract**

The heating packet of the present invention comprises an air-permeable bag containing a heat-generating composition or a sheet-like heat-generating body which generates heat upon contacting oxygen in air. The heat-generating composition or the sheet-like heat-generating body contains zinc powder instead of iron powder. By the use of zinc powder, the heat-generating composition and the sheet-like heat-generating body can be maintained flexible during the use of the heating packet or after heat generation.

## Description

### BACKGROUND OF THE INVENTION

### 1. Field of the Invention

The present invention relates to a heating packet, specifically, to a heating packet comprising an air-permeable bag containing a heat-generating composition or a sheet-like heat-generating body which generate heat upon contacting oxygen in air, and more specifically, to a heating packet which remains flexible even after the heat generation is ceased.

### 2. Description of the Prior Art

Heating packets, which comprise an air-permeable bag containing a heat-generating composition mainly comprising powder of oxidizable metal, activated carbon, an inorganic electrolyte and water and evolving heat upon contacting oxygen in air, has been widely used in various applications. These heating packets are generally produced by packing a wet, powdery heat-generating composition prepared by mixing an iron powder, activated carbon, an inorganic electrolyte, water, etc. into a flat air-permeable bag. Alternatively, in stead of the powdery heat-generating composition, a sheet-like heat-generating body may be packed into a flat air-permeable bag. The sheet-like heat-generating body is prepared by supporting the heat-generating composition on a sheet-like body, such as non-woven fabric, having a number of voids.

The heating packet is sealed and stored in an air-impermeable outer bag before use to keep the heating packet away from contacting the surrounding air and prevent the water in the heating packet from escaping into the surrounding air.

Also known is a so-called adhesive heating packet in which one surface of the flat bag is made air-permeable and the other surface is partly or entirely made into a non-transferring adhesive or sticky surface. The adhesive heating packet is attached to a body garment through the non-transferring adhesive or sticky surface to facilitate the attachment of the heating packet to the physical body.

To improve the adhesive heating packet by more efficiently conducting the heat from the heating packet to the physical body and reducing the weight and thickness of the heating packet, developed is a so-called next-to-skin heating packet in which the flat bag has an air-permeable surface on one side and an adhesive surface on the other side and the heating packet is directly attached to the body skin through the adhesive surface.

These known heating packets, adhesive heating packets and next-to-skin heating packets have been used to warm the physical body against the cold, to prevent overcooling of the physical body due to air-cooling in summer, to relieve or remove the pain such as physiological pain, neuralgic pain, muscular pain, etc., or to relieve physical fatigue.

U-shaped and toe-shaped heating packet for placing in a shoe have been developed and put on the market. In addition, a purse-shaped heating packet is also commercially available.

However, the known heating packets have the following defects.

The heat-generating compositions used in the known heating packets are gradually hardened to produce a straggle of gravel-like solids with the progress of the exothermic reaction, or become a hard solid plate at the final stage of the exothermic reaction, although the heat-generating compositions are flowable, wet powder before use. Particularly, when the heating packet is kept stationary during its use without deformation or movement, the heat-generating composition is entirely hardened into a single solid plate, thereby bringing the users discomfort, preventing an efficient transfer of heat from the heating packet to the physical body due to failure in closely contacting the body contours and giving uncomfortable rigid feeling to the users.

Like the known heat-generating compositions, the known sheet-like heat-generating body is gradually hardened into a rigid plate during the use, although it is flexible before use.

Since directly attached to the body skin, the known next-to-skin heating packet gives a severe uncomfortable feeling to the users when it becomes rigid during the use and the heating packet attached to the body skin sometimes falls off in a short use period. Also, if the heating packet is held under thin clothes, the use of the heating packet is likely to be recognized from outside due to its plate-like shape.

If the heat-generating composition of the known heating packet for use in footwear is displaced unevenly during the use and hardened as such, an uncomfortable feeling due to rigid solids is given to the users at the toes, thereby restricting the walking.

When the purse-shaped heating packet is used in a stationary state, the entire portion of the heat-generating composition is hardened after the heat generation to form a rigid solid like a stone.

### SUMMARY OF THE INVENTION

In view of the above problems, an object of the present invention is to provide a heating packet free from the hardening of the heat-generating composition or the sheet-like heat-generating body during its use, thereby avoiding uncomfortable feeling due to rigid solids, preventing the attached heating packet from falling off, and ensuring a sufficient transfer of heat to the physical body.

As a result of intensive study in view of solving the above problems, the inventors have found that a heat-generating composition or a sheet-like heat-generating body containing zinc powder instead of iron powder exhibits excellent heat generation properties and that the above problems can be solved by such a heat-generating composition or a sheet-like heat-generating body because they are not hardened during heat generation and even after heat generation. The present invention has been accomplished based on these findings.

Thus, the present invention provides a heating packet for heating physical body, comprising an air-permeable bag containing a heat-generating composition or a sheet-like heat-generating body which generates heat upon contacting oxygen in air, the heat-generating composition or the sheet-like heat-generating body containing zinc powder.

The heating packet of the present invention comprises a powdery heat-generating composition or a sheet-like heat-generating body, each containing a zinc powder as its component, packaged in an air-permeable bag. The heating packet of the present invention utilizes the heat generation during the formation of zinc oxide and zinc hydroxide by the reaction of zinc with water and oxygen in air or the formation of a salt of zinc with an additive to the heat-generating composition. The inventors have found a method for efficiently oxidizing zinc by bringing zinc into contact with oxygen in air and further found that the heat-generating composition and the sheet-like heat-generating body are not hardened even after the oxidation of zinc. With such findings, it has made possible to provide an ever-flexible heating packet capable of efficiently transferring the heat to the physical body without uncomfortable feeling during its use.

### BRIEF DESCRIPTION OF THE DRAWINGS

Fig. 1 is a sectional view showing a heating packet of the present invention, in which a powdery heat-generating composition is packaged in a flat bag;
Fig. 2 is a sectional view showing another heating packet of the present invention, in which a sheet-like heat-generating body is packaged in a flat bag; and
Fig. 3 is a sectional view showing a purse-shaped heating packet containing a powdery heat-generating composition.

### DETAILED DESCRIPTION OF THE INVENTION

The heat-generating composition used in the present invention comprises, as the major components, a zinc powder, a salt and water.

The zinc powder used in the heating packet of the present invention is a powder of zinc metal. The production method thereof is not strictly limited, and the zinc powder produced by electrolysis, vertical distillation, electrically heated distillation, ISP method, etc. may be usable. The zinc powder may be purified as high as about 99%, or may contain impurities such as oxides and hydroxides of zinc in an amount not adversely affect the heat generation by the contact with oxygen in air. The particle size of the zinc powder is not particularly limited, and preferably 0.005 to 1.5 mm, more preferably 0.05 to 0.75 mm, and most preferably 0.15 to 0.5 mm.

The salt used in the present invention is a neutral, weakly acidic or weakly basic inorganic or organic salt. The neural salt may be a halide, sulfate or phosphate of alkali metal such as sodium chloride, potassium chloride, sodium sulfate and sodium orthophosphate. The weakly acidic salt may include ammonium chloride, zinc chloride, iron(III) chloride, etc. The weakly basic salt may include sodium carbonate, potassium carbonate, potassium hydrogencarbonate, sodium oxalate, sodium acetate, etc. These salts may be used alone or in combination of two or more.

The mixing ratio of the zinc powder, water and the salt in the heat-generating composition is not particularly specified because it may vary depending on the intended heat-generating properties, the presence or absence of the auxiliary additive such as a reaction aid, a water retainer, etc. mentioned below and its addition amount. Water is preferably used in an amount of 10 to 100 parts by weight, more preferably 25 to 70 parts by weight based on 100 parts by weight of the zinc powder. The salt is preferably used in an amount of 0.2 to 50 parts by weight, more preferably 1 to 30 parts by weight based on 100 parts by weight of the zinc powder.

The heat-generating composition of the present invention may further contain, if desired, an activated carbon as a reaction aid and a water retainer. The activated carbon as the reaction aid also serves as a water retainer, and may include a coconut shell activated carbon, a wood activated carbon and a peat activated carbon. Substances having a high water retention and causing no degradation during a long-term storage of the heating packet may be used as the water retainer without no particular limitation. Examples thereof include a pearlite powder, vermiculite, wood powder, a water-absorbing polymer, etc. Each of the reaction aids and the water retainers may be used alone or in combination of two or more. The addition amount thereof is not particularly limited.

Another heat-generating composition usable in the present invention comprises the zinc powder, water and an alkaline substance such as a hydroxide of alkali metal or alkaline earth metal and an amine, more particularly, sodium hydroxide, potassium hydroxide, calcium hydroxide, barium hydroxide, t-butylhydrazine, etc. These alkaline substances may be used alone or in combination of two or more.

The mixing ratio of the zinc powder, water and the alkaline substance in the heat-generating composition is not particularly specified because it may vary depending on the intended heat-generating properties, the presence or absence of the auxiliary additive such as a reaction aid, a water retainer, etc. and its addition amount. Preferably, water is used in an amount of 15 to 100 parts by weight, more preferably 30 to 70 parts by weight based on 100 parts by weight of the zinc powder. The addition amount of the alkaline substance is preferably 0.2 to 50 parts by weight, more preferably 1 to 20 parts by weight based on 100 parts by weight of the zinc powder.

In addition to the zinc powder, water and the alkaline substance, the salt described above may be added to the heat-generating composition preferably in an amount of 0.2 to 50 parts by weight, more preferably 1 to 20 parts by weight based on 100 parts by weight of the zinc powder. Further, an additive such as the activated carbon and the water retainer may be added to the heat-generating composition.

The heating packet of the present invention may comprises an air-permeable bag containing, instead of the powdery heat-generating composition described above, a sheet-like heat-generating body which is prepared by supporting a mixture comprising the zinc powder, water and at least one of the salt and the alkaline substance on a sheet-like body. The structure and the production method of the sheet-like heat-generating body are not particularly limited and may be produced by methods known in the art.

For example, the sheet-like heat-generating body can be produced by the following methods:
(1) The zinc powder is supported throughout a sheet-like support comprising randomly arranged fibers and having numerous voids inside thereof. Then, the sheet-like support is impregnated with an aqueous solution of at least one of the salt and the alkaline substance.
(2) A porous non-woven fabric A having numerous voids is adhesively bonded to a non-woven fabric B. The zinc powder or a mixture of the zinc powder and at least one of the salt and the alkaline substance is sprinkled over the porous non-woven fabric A to hold them into the voids. Then, a non-woven fabric C is laminated on the porous non-woven fabric Aby heat compression using an embossing machine, followed by the impregnation of the resultant laminate with an aqueous solution of at least one of the salt and the alkaline substance, or water.

In addition, usable is a sheet-like heat-generating body produced by supporting the zinc powder, water and at least one of the salt and the alkaline substance on a sheet-like body during the formation of the sheet-like body by a paper machine. Also usable is a sheet-like heat-generating body produced by supporting the zinc powder, water and at least one of the salt and the alkaline substance on a flexible, porous sheet-like body made of a foamed plastic, a reticulated material, etc.

The mixing ratio of the zinc powder, water and the salt, or the mixing ratio of the zinc powder, water and the alkaline substance is substantially the same as in the case of the powdery heat-generating composition mentioned above. In addition, the activated carbon and the water retainer may be used in an amount depending on the water retention, hydrophilic nature, air permeability, etc. of the non-woven material or the porous material constituting the sheet-like body.

The air-permeable bag used in the present invention is not specifically limited as far as it is air-permeable enough to pass a sufficient amount of oxygen for oxidizing zinc without causing breaking, leaking of the heat-generating composition or the sheet-like heat-generating body and plastic deformation during its use. Also, the material for the bag, the shape of permeable pores and the size of permeable pores are not critical in the present invention. The dimension and the shape of the bag are also not critical, and the bag may be made into a flat bag having a rectangular shape, a circular shape, an animal shape, a vegetable shape or a doll shape. The bag may be made into U-shape, toe-shape or elliptical shape when a heating packet for placing in a footwear is intended. In addition, the bag may be made into a purse shape.

The dimension of the heating packet is not particularly limited. When the heating packet is flat, the area of one surface is preferably 5 to 5000 cm², more preferably 20 to 2500 cm².

The heating packet may be made air-permeable in its entire surface or partial surface. For example, in the case of a flat heating packet, only one surface or both surfaces can be made air-permeable in its entire or partial portion.

Examples of the air-permeable material for the bag include a non-woven fabric, a woven fabric, a paper, a porous film having numerous minute open pores, an air-permeable laminate produced by making an air-impermeable laminate of a non-woven or woven fabric and a polyethylene film, etc. air-permeable by providing minute openings, a laminate of a non-woven fabric and a porous film, and a non-woven fabric having an air permeability controlled by heat-pressing a matting of fibers.

The porous film referred to above is a film containing minute open pores having a maximum pore size of about 0.01 to 20 µm when measured by a methanol bubbling method. The porous film is produced, for example, by biaxially stretching a synthetic resin film or by stretching a film obtained by extruding a melt polyethylene, polypropylene, etc. containing dispersed inorganic fine powder such as calcium carbonate. The porous film is commercially available as Porum and NF Sheet (both trade name of Tokuyama Co., Ltd.), Cellpore (trade name of Sekisui Chemical Co., Ltd.) and Breathron (trade name Nitto Denko Co., Ltd.). The non-woven fabric having an air-permeability controlled by heat-pressing a matting of fibers is commercially available as Tyvek (trade name of Du Pont).

The permeability is generally expressed by a gas transmission rate of JIS P8117, a gas permeability of JIS L1018 or a water vapor transmission rate of JIS Z0208. However, the permeability determined by these methods are not necessarily in direct correlation with the heat generating properties depending on the permeable pore size, the pressure difference between the opposite surfaces of the air-permeable material for the bag during the measurement and the hygroscopic nature of the air-permeable material for the bag. Therefore, in the present invention, the air permeability is expressed by the oxygen diffusion rate proposed by the inventors in Japanese Patent Application Laid-Open No. 11-30579.

The oxygen diffusion rate referred to in the present invention is the amount of oxygen diffused across the air-permeable material for the bag from the atmosphere into a carrier gas when measured under conditions of 20°C, 65% relative humidity and atmospheric pressure by exposing one surface of the air-permeable material to the atmosphere while sweeping the opposite surface with the oxygen-free nitrogen carrier gas at a flow rate of 0.193 M/cm²/h (normal liter/square centimeter/hour) per unit surface area of the air-permeable material.

Since the pressure difference between the atmospheric side and the carrier gas side is kept substantially zero during the measurement, the measured value is less affected by the permeable pore size. In addition, the measurement is carried out by exposing one side to the atmosphere while maintaining the other side (carrier gas side) extremely low in the oxygen concentration as compared with the atmosphere, i.e., while maintaining the other side in nearly the same condition with respect to oxygen concentration as the inside of the heating packet during its use. Therefore, the measured permeability is well related to the heat generation properties.

The air permeability of the heating packet of the present invention is selected depending on the mode of use such as a heating packet for holding on the physical body, a heating packet for placing in footwear, a heating packet of purse-like shape, etc, the shape of heating packet and the intended heat generation properties. In the case of a flat heating packet for holding on the physical body having an air-permeable surface only on one side, the air permeability in terms of the oxygen diffusion rate is preferably 450 to 1500 Nl/m²/24h (normal liter/square meter/24 hours), more preferably 600 to 1200 Nl/m²/24h. The heat generation is poor when the oxygen diffusion rate is less than 450 Nl/m²/24h, while overheating of the body skin is caused when larger than 1500 Nl/m²/24h.

When both the surfaces of a flat heating packet are made air-permeable, the air permeability of each surface of the heating packet is determined in consideration of the decrease in the air permeability of the surface facing the body skin so that the average of the air permeabilities of both the surfaces falls within 300 to 1000 Nl/m²/24h.

In the case of the heating packet for placing in footwear having an air-permeable surface only on one side, the air permeability in terms of the oxygen diffusion rate is preferably 4400 to 6600 Nl/m²/24h.

When both the surfaces are made air-permeable, the air permeability of each surface is determined in consideration of the decrease in the air permeability of the surface facing the inner surface of the footwear so that the average of the air permeabilities of both the surfaces falls within 2000 to 4000 Nl/m²/24h.

In the case of the purse-shaped heating packet having uniform air permeability throughout the entire surface, the air permeability in terms of the oxygen diffusion rate is preferably 1000 to 2000 Nl/m²/24h.

A non-transferring adhesive portion can be formed on the heating packet so as to facilitate the attachment of the heating packet to the physical body. Any adhesive is usable as far as it has a sufficient adhesive force, it is not transferred to body garments, it is not detrimental to human health and environment, and it is not degraded during a long-term storage before use. For example, the adhesive based on acrylic polymer, rubber polymer or vinyl acetate polymer is preferably used. The surface of the adhesive portion is covered with a releasing paper before use to avoid undesirable sticking.

The flat heating packet of the present invention in which only one air-permeable surface is provided with an adhesive portion can be attached to the inside of body garment via the air-permeable, adhesive surface. In this mode of use, the adhesive is prevented from directly contacting the body skin while the heating packet is allowed to contact the body skin. Therefore, itch and eruption on the skin contacting the adhesive, which are inevitable drawback of the next-to-skin heating packet, can be avoided. Also, since the air permeability can be kept constant during its use in any posture or body movements, heat is generated stably.

The area ratio of the adhesive portion, if provided, is not critical in the present invention. Also, the adhesive portion is made into any pattern such as polka dots, spots, honeycomb, stripe and check.

The heating packet of the present invention can be made antibacterial. The antibacterial agent is not specifically limited in its kind, its form and the method of mixing. Any antibacterial agent can be used as far as it is not detrimental to human health and environment, and it does not adversely affect the heat generation properties of the heating packet. For example, an inorganic antibacterial agent such as heavy metal and its salt, a quaternary ammonium antibacterial agent, a guanidine antibacterial agent, a phenol antibacterial agent and a natural antibacterial agent are preferably used.

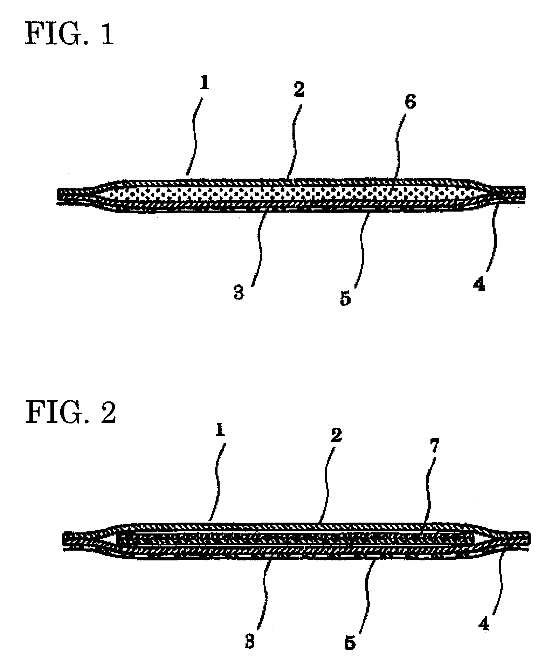

The present invention will be described by referring to Figs. 1 to 3 which are not intended to restrict the scope of the present invention thereto.

Fig. 1 is a sectional view showing a heating packet 1 of the present invention. Apowdery heat-generating composition 6 is packaged in a flat bag which is made from a air-permeable material 2 and an air-impermeable material 3 which are heat-sealed at their peripheries 4.

Fig. 2 is a sectional view showing another heating packet 1 of the present invention. A sheet-like heat-generating body 7 is packaged in a flat bag similar to the bag shown in Fig. 1.

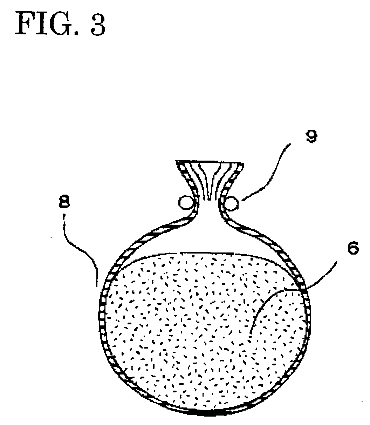

Fig. 3 is a sectional view showing a purse-shaped heating packet 8 containing a powdery heat-generating composition 6. The opening of the purse-shaped heating packet is closed by a clip 9.

The present invention will be described in further detail by way of the following examples. However, it should be noted that the following examples are illustrative and not intended to limit the present invention thereto.

### EXAMPLE 1

A powdery heat-generating composition was prepared in nitrogen atmosphere by mixing 20 g of zinc powder, 9 g of water, 3 g of sodium hydroxide, 4 g of wood powder and 1.5 g of water absorbing polymer.

The oxygen diffusion rate of a porous laminate of a nylon non-woven fabric and a porous polyethylene film (Breathron available from Nitto Denko Co., Ltd.) was measured at 20°C and 65% relative humidity while exposing the nylon side to the atmosphere and sweeping the other side with nitrogen flow at a flow rate of 0.193 Nl/cm²/h. The measured oxygen diffusion rate was 810 Nl/m²/24h.

Separately, a nylon non-woven fabric having a basis weight of 45 g/m² and a polyethylene film having a thickness of 30 µm were laminated. On the nylon non-woven fabric of the laminate, circular adhesive portions of 6 mm diameter were formed at 10 mm intervals in both the longitudinal and transverse directions. The adhesive portions were covered with a releasing paper, thereby obtaining an adhesive laminate.

The porous laminate and the adhesive laminate each cut out into a size of 135 mm long and 100 mm wide were stacked so that two polyethylene films faced to each other. Then, the stacked laminate was heat-sealed at its three sides in 5 mm sealing width to prepare a flat bag.

After placing the heat-generating composition into the flat bag, the open side of the bag was heat-sealed to obtain a heating packet of so-called adhesive type. Five heating packets were sealed in respective air-impermeable outer packages.

The heat generation properties of the heating packet taken out of the outer package were measured using a temperature characteristic tester of JIS S4100. As a result thereof, the heating packet was confirmed to have good heat generation properties as evidenced by that the time required to reach 40°C (rise time) was 9 minutes, the ultimate highest temperature was 57°C and the duration of maintaining at 40°C or higher (duration time) was 13.2 hours. In addition, the heating packet remained flexible even after heat generation, and no hardened mass was observed in the heat-generating composition.

### EXAMPLE 2

In the same manner as in Example 1 except for using a heat-generating composition consisting of 20 g of zinc powder, 8.2 g of water, 1 g of sodium chloride, 3 g of wood powder, 1.5 g of water-absorbing polymer and 2.5 g of activated carbon, five heating packets were prepared.

The results of the same measurement of the heat generation properties as in Example 1 showed that the heating packet had good heat generation properties of a rise time of 7 minutes, an ultimate highest temperature of 56°C and a duration time of 12.8 hours. In addition, the heating packet remained flexible even after heat generation, and no hardened mass was observed in the heat-generating composition.

### EXAMPLE 3

In the same manner as in Example 1 except for using a heat-generating composition consisting of 20 g of zinc powder, 8.2 g of water, 1 g of sodium chloride, 1.0 g sodium hydroxide, 3 g of wood powder, 0.34 g of water-absorbing polymer and 2.5 g of activated carbon, heating packets were prepared.

The results of the same measurement of the heat generation properties as in Example 1 showed that the heating packet had good heat generation properties of a rise time of 8 minutes, an ultimate highest temperature of 57°C and a duration time of 13 hours. In addition, the heating packet remained flexible even after heat generation, and no hardened mass was observed in the heat-generating composition.

### EXAMPLE 4

On a wood pulp non-woven fabric having a basis weight of 40 g/m² and a size of 120 mm length and 90 mm width, a wood pulp non-woven fabric having a basis weight of 60 g/m² with the same dimension was stacked after wetting the bottom surface with water. A mixture of 10 g of zinc powder, 0.2 g of water-absorbing polymer, 1.25 g of activated carbon and 0.2 g of powdery ethylene-vinyl acetate copolymer was sprinkled over the surface of the resultant stack. The stack was vibrated by a vibrator to allow the sprinkled mixture to enter into the voids of the non-woven fabric. Then, a wood pulp non-woven fabric having a basis weight of 40 g/m² with the same dimension was stacked on the sprinkled surface. The resultant stack was formed into a sheet by passing it through embossing rolls heated to 200°C. Finally, 4.6 g of 11 weight % aqueous solution of sodium chloride were sprinkled over the sheet to obtain a sheet-like heat-generating body.

The sheet-like heat-generating body obtained above was placed in a flat bag prepared in the same manner as in Example 1 to obtain a heating packet, which was then sealed in an air-impermeable outer package.

The results of the same measurement of the heat generation properties as in Example 1 showed that the heating packet had good heat generation properties as evidenced by a rise time of 10 minutes, an ultimate highest temperature of 52°C and a duration time of 7.5 hours. In addition, the heating packet remained flexible even after heat generation, and no hardening was occurred.

### EXAMPLE 5

A heat-generating composition was prepared in nitrogen atmosphere by mixing 25 g of zinc powder, 10.3 g of water, 1.25 g of sodium chloride, 1.25 g of sodium hydroxide, 3.75 g of wood powder, 0.42 g of water absorbing polymer and 3.2 g of activated carbon.

Separately, on a nylon non-woven fabric (210 mm x 210 mm) having a basis weight of 30 g/m², a porous polyethylene film having the same dimension was stacked and spot-bonded to the non-woven fabric through the spots (1 mm diameter) of a hot-melt adhesive resin provided one per 1 cm² of the bonding surface, thereby obtaining an air-permeable material.

The oxygen diffusion rate of the air-permeable material was measured at 20°C and 65% relative humidity while exposing the nylon side to the atmosphere and sweeping the other side with nitrogen flow at a flow rate of 0.193 M/cm²/h. The measured oxygen diffusion rate was 1400 Nl/m²/24h.

The heat-generating composition prepared above was wrapped by the air-permeable material with the porous polyethylene layer inside. After closing the opening by a clip, the gathered air-permeable material over the clip was cut off to obtain a purse-shaped heating packet as shown in Fig. 3. The obtained heating packet was sealed in an air-impermeable outer package.

The heat generation properties of the heating packet taken out of the outer package was measured in the same manner as in Example 1. The results showed that the rise time was 7 minutes, the ultimate highest temperature was 63°C and the duration time was 8 hours. In addition, the heating packet remained flexible even after heat generation, and no hardened mass was observed in the heat-generating composition.

### COMPARATIVE EXAMPLE 1

Aheating packet was prepared in the same manner as in Example 1 except for using a heat-generating composition consisting of 20 g of iron powder, 2.5 g of activated carbon, 3 g of wood powder, 0.4 g of water-absorbing polymer, 1 g of sodium chloride and 8 g of water.

The heat generation properties of the heating packet were measured in the same manner as in Example 1. The results showed that the rise time was 8 minutes, the ultimate highest temperature was 56°C and the duration time was 12.5 hours. However, the heat-generating composition after heat generation was hardened into a rigid solid plate.

Since the powdery heat-generating composition and the sheet-like heat-generating body of the present invention are not hardened into a rigid mass, the heating packet which remains flexible during its use can be provided. With the flexibility throughout its use, the heating packet is kept in good contact with the body contours, thereby ensuring the efficient heat transfer and eliminating uncomfortable rigid feeling during its use. Since the uncomfortable feeling at the toes due to the hardening of the heating packet for footwear can be avoided, the walking is not restricted even when the heating packet is kept placed in footwear. In the case of a so-called next-to-skin type heating packet which is directly attached to the body skin, the uncomfortable rigid feeling and the falling off of the attached heating packet are avoided. In addition, the use of the heating packet is hardly recognized even through thin clothes.

## Claims

1. Aheating packet for heating physical body, comprising an air-permeable bag containing a heat-generating composition or a sheet-like heat-generating body which generates heat upon contacting oxygen in air, the heat-generating composition or the sheet-like heat-generating body containing zinc powder.

2. The heating packet according to claim 1, wherein the heat-generating composition contains at least the zinc powder, water and a salt.

3. The heating packet according to claim 1, wherein the sheet-like heat-generating body comprises a non-woven fabric having numerous voids or a flexible porous body each supporting at least the zinc powder, water and a salt.

4. The heating packet according to claim 2 or 3, wherein the salt is a neutral salt, a weakly acidic salt or a weakly basic salt.

5. The heating packet according to any one of claims 2 to 4, wherein the amount of water is 10 to 100 parts by weight and the amount of the salt is 0.2 to 50 parts by weight each based on 100 parts by weight of the zinc powder.

6. The heating packet according to claim 1, wherein the heat-generating composition contains at least the zinc powder, water and an alkaline substance.

7. The heating packet according to claim 1, wherein the sheet-like heat-generating body comprises a non-woven fabric having numerous voids or a flexible porous body each supporting at least the zinc powder, water and an alkaline substance.

8. The heating packet according to claim 6 or 7, wherein the alkaline substance is a hydroxide of alkali metal, a hydroxide of alkaline earth metal or an amine.

9. The heating packet according to any one of claims 6 to 8, wherein the amount of water is 15 to 100 parts by weight and the amount of the alkaline substance is 0.2 to 50 parts by weight each based on 100 parts by weight of the zinc powder.

10. The heating packet according to any one of claims 1 to 9, wherein the heating packet has a first surface and a second surface opposite to the first surface, one of the first surface and the second surface being made adhesive.

11. The heating packet according to any one of claims 1 to 10, wherein the air-permeable bag is a flat bag having a first surface and a second surface opposite to the first surface, and wherein one of the first surface and the second surface comprises an air-permeable material having an air permeability of 450 to 1500 Nl/m²/24h in terms of an oxygen diffusion rate which is defined as an amount of oxygen diffused across the air-permeable material when measured at 20°C and 65% relative humidity under atmospheric pressure by exposing one of the sides of the air-permeable material to the atmosphere while sweeping the other side with an oxygen-free nitrogen gas flow at a flow rate of 0.193 Nl/cm²/h per unit area of the air-permeable material.

12. The heating packet according to any one of claims 1 to 10, wherein the air-permeable bag is a flat bag having a first surface and a second surface opposite to the first surface, and wherein both the first surface and the second surface comprises air-permeable materials and an average air permeability of the first surface and the second surface is 300 to 1000 Nl/m²/24h in terms of an oxygen diffusion rate which is defined as an amount of oxygen diffused across the air-permeable material when measured at 20°C and 65% relative humidity under atmospheric pressure by exposing one of the sides of the air-permeable material to the atmosphere while sweeping the other side with an oxygen-free nitrogen gas flow at a flow rate of 0.193 Nl/cm²/h per unit area of the air-permeable material.

13. The heating packet according to any one of claims 1 to 10, wherein the air-permeable bag is a flat bag having a first surface and a second surface opposite to the first surface, and wherein one of the first surface and the second surface comprises an air-permeable material having an air permeability of 4400 to 6600 Nl/m²/24h in terms of an oxygen diffusion rate which is defined as an amount of oxygen diffused across the air-permeable material when measured at 20°C and 65% relative humidity under atmospheric pressure by exposing one of the sides of the air-permeable material to the atmosphere while sweeping the other side with an oxygen-free nitrogen gas flow at a flow rate of 0.193 Nl/cm²/h per unit area of the air-permeable material.

14. The heating packet according to any one of claims 1 to 10, wherein the air-permeable bag is a flat bag having a first surface and a second surface opposite to the first surface, and wherein both the first surface and the second surface comprises air-permeable materials and an average air permeability of the first surface and the second surface is 2000 to 4000 Nl/m²/24h in terms of an oxygen diffusion rate which is defined as an amount of oxygen diffused across the air-permeable material when measured at 20°C and 65% relative humidity under atmospheric pressure by exposing one of the sides of the air-permeable material to the atmosphere while sweeping the other side with an oxygen-free nitrogen gas flow at a flow rate of 0.193 Nl/cm²/h per unit area of the air-permeable material.

15. The heating packet according to any one of claims 1 to 10, wherein the air-permeable bag is made of an air-permeable material having an air permeability of 1000 to 2000 Nl/m²/24h in terms of an oxygen diffusion rate which is defined as an amount of oxygen diffused across the air-permeable material when measured at 20°C and 65% relative humidity under atmospheric pressure by exposing one of the sides of the air-permeable material to the atmosphere while sweeping the other side with an oxygen-free nitrogen gas flow at a flow rate of 0.193 Nl/cm²/h per unit area of the air-permeable material.
